# EUROPEAN PATENT SPECIFICATION

(11) **EP 1 489 345 B1**
(45) Date of publication and mention of the grant of the patent: **01.03.2006**
(21) Application number: 03425391.4
(22) Date of filing: 16.06.2003
(51) Int. Cl.: F16L 37/40, F16L 37/413, F16L 37/23

(54) **Supplying terminal unit for systems distributing compressed gas**
Versorgungsstutzeneinheit für ein Druckgasverteilungssystem
Embout de distribution pour un système de distribution pour gaz comprimé

(43) Date of publication of application: 22.12.2004
(73) Proprietor: Flow Meter S.p.a., 24040 Levate (Bergamo) (IT)
(72) Inventor: Paratico, Roberto c/o Flow-Meter S.p.A., 24040 Levate (IT)
(74) Representative: Botti, Mario

(56) References cited:
- DE-A- 2 741 512
- DE-U- 8 527 018

## Description

### Field of application

The present invention, in its most general aspect, relates to a distribution plant of compressed gases, indicatively and not limitedly for compressed gases for medical use, such as medicinal gases and hospital technical gases.

In particular, this invention relates to a supplying terminal unit of such a distribution plant: such a terminal unit, which is substantially a delivering group, comprises a socket, which is generally fixed to a wall of a room, for example in a wall box (alternatively, the socket can be joined to a flexible piping to supply consumptions), and which is associated with a compressed gas source, and a joint, or plug, arranged at an end of a compressed gas consumption piping and which is in fluid communication with such piping, an operator performing connections and disconnections of the joint from the socket on said unit, with simultaneous and respective deliveries and interruptions of the gas flow in the consumption piping.

It is to be specified that, in the present application, in general medicinal gases mean single gases or gas mixtures which have curative or prophylactic properties for human illnesses and are intended to be administered to a person in order to make a medical diagnosis or to restore, correct or modify person's physical functions. Moreover, - hospital technical gases mean -single gases or those gas mixtures which are used in the hospital environment for non-therapeutic purposes, such as diagnostic, apparatus calibration, surgical instrument actuation, etc. purposes.

Of course, all that is hereinafter described and claimed for a supplying terminal unit for compressed gas distribution plant , is also valid in the case of an extractor terminal unit for a so-called vacuum distribution plant, where the analogies with the supplying terminal unit, which is given as a non-limiting example , are clear.

### Prior art

As it is known, the socket is connected to the gas distribution plant and includes, at an end of a socket body associated with a compressed gas source, a valve seat in fluid communication with the distribution plant, being foreseen a shutter of the valve seat stimulated to close by elastic means. The socket also includes a seat, generally coaxial and contiguous to the valve seat and, therethrough, in fluid communication with the compressed gas source.

The joint can be inserted in said seat up to a delivery position, in such a position a free end of said joint is active on the shutter and keeps it opening the respective valve seat. Removable blocking means of the joint in said socket are simultaneously provided.

More specifically, the joint is a component which, inserted in the socket, remains blocked therein at the same time as the delivery of the gas.

After the use of the piping with a determined consumption, the joint is unblocked and released from the socket by an operator, such an operation also involving the instantaneous and necessary interruption of the gas supply, which takes place through the shutter which, under the action of the elastic means on which it is mounted, closes the valve seat.

However, such a releasing operation has the great drawback that the operator must be particularly careful with it, since the compressed gases present in the consumption piping can determine dangerous situations for the operator himself: consider, for example, that the gases used to actuate the surgical tools have pressures of about 800 kPa and therefore the piping - generally flexible tubes - can become actual whips, with a violent projection of the joint disconnected in the direction of the operator.

To operate in safety, during disconnection, the operator is taught to pay great attention and preferably to use both hands, one to disconnect the joint and the other to support the end part of the consumption piping, so that the joint cannot be projected from the compressed gas.

A joint having one end associated with a consumption piping of said compressed gas, and the other end free,
a socket having a socket body associated with a source of said compressed gas,
a seat defined in said socket body and in fluid communication with said compressed gas source,
a gas interception valve, including a valve seat formed in said body, open in said seat, and a shutter stimulated to close said valve seat by elastic means, is known from DE 2741512.

### Summary of the invention

The problem underlying the present invention is that of providing a supplying terminal unit, for distribution plants of compressed gases, able to overcome, in a simple and economical way, all of drawbacks quoted with reference to the prior art.

This problem is solved, according to the present invention, by a supplying terminal unit for distribution plants of compressed gases characterized in that it comprises:
a sleeve movably guided in the seat of said socket body, between an operative position, near to said valve seat and a non-operative position, spaced from said valve seat,
first removable blocking means of said joint in said sleeve, said first blocking means being removable only when said sleeve is in non-operative position,
second removable blocking means of said sleeve in said seat, which act when said first blocking means of said joint in said sleeve are active and when said sleeve is in operative position, in a position wherein the free end of said joint is active on said shutter and keeps it opening the respective valve seat.

The advantages and characteristics of the supplying terminal unit for distribution plants of compressed gases for medical use, according to the present invention, will become clearer from the description of an embodiment thereof, made hereinafter with reference to the attached drawings given for indicative and not limiting purposes.

### Brief description of the drawings

Figure 1 schematically shows a middle section view of a supplying terminal unit according to the invention, comprising a socket and a joint, where the joint is disconnected from the socket.
Figure 2 schematically shows a middle section view of the supplying unit of figure 1, in a blocking position of the joint in the socket without fluid delivery.
Figure 3 schematically shows a middle section view of the supplying unit of figure 1, in a further blocking position of the joint in the socket with fluid delivery.

### Detailed description of a preferred embodiment

With reference to the figures, a supplying terminal unit for distribution plants of compressed gases in accordance with the present invention is shown and is globally indicated with 9.

Said supplying terminal unit 9 comprises a socket 10 and a joint 16.

The socket 10 includes, in a socket body 27 associated with a compressed gas source, a seat 14 defined in said socket body 27 and in fluid communication with the compressed gas source.

More specifically, the seat 14 is in fluid communication with a gas interception valve 18, including a valve seat 19, formed in said body 27 and open in said seat 14, and a shutter 12 stimulated to close said valve seat 19 by elastic means 13, such as a helical spring.

The joint 16, generally tubular, has one end 17 associated with a consumption piping of said compressed gas, not shown in the figures, and the other end 20 free.

Preferably, the socket 10 has an axial symmetry with respect to an axis Z-Z and such an axis coincides with that of the seat 14.

The opening of the valve seat 19 takes place when the shutter 12 is displaced towards the elastic means 13 whereon it is mounted by the free end 20 of the joint 16 when it has been inserted, for a predetermined stroke along the axis Z-Z, within the seat 14.

More precisely, the joint 16 is taken up to a blocking and delivery position where blocking means 22 of the joint 16 in the seat 14 act at the same time as the opening of the valve seat 19.

The blocking means 22 are, in the example of the figures, of the release type, for example they provide a series of balls 24 distributed circumferentially around the axis Z-Z, such balls 24 moving in directions substantially perpendicular to the axis Z-Z between recesses 26, integral with the socket body 27 of the socket 10, and throats 28, integral with the joint 16: when the joint 16 is in the blocking position, the balls 24 are arranged within the throats 28 integral with the joint 16.

In accordance with an aspect of the present invention, the socket 10 comprises further removable blocking means 30 of the joint 16 in the seat 14, which advantageously act when the aforementioned blocking means 22 of the joint 16 are disengaged.

Such removable blocking means 30 are preferably mounted on a maneuvering equipment 32, with which the socket 10 is provided.

The maneuvering equipment 32 is axially mobile along the axis Z-Z of the socket 10 and comprises a sleeve 40, mounted coaxially to the axis Z-Z of the socket 10 and inside the seat 14, an inner bush 34 and an outer bush 36, mounted coaxially to the axis Z-Z of the socket 10 and externally around the seat 14.

More precisely, the inner bush 34 can be axially displaced within the outer bush 36, whereas the outer bush 36 can be axially displaced with respect to the socket body 27 of the socket 10.

The sleeve 40 is mounted integral with and inside the outer bush 36, the sleeve 40 also being inside the inner bush 34: such a sleeve 40, at an end, constitutes a support for the joint 16.

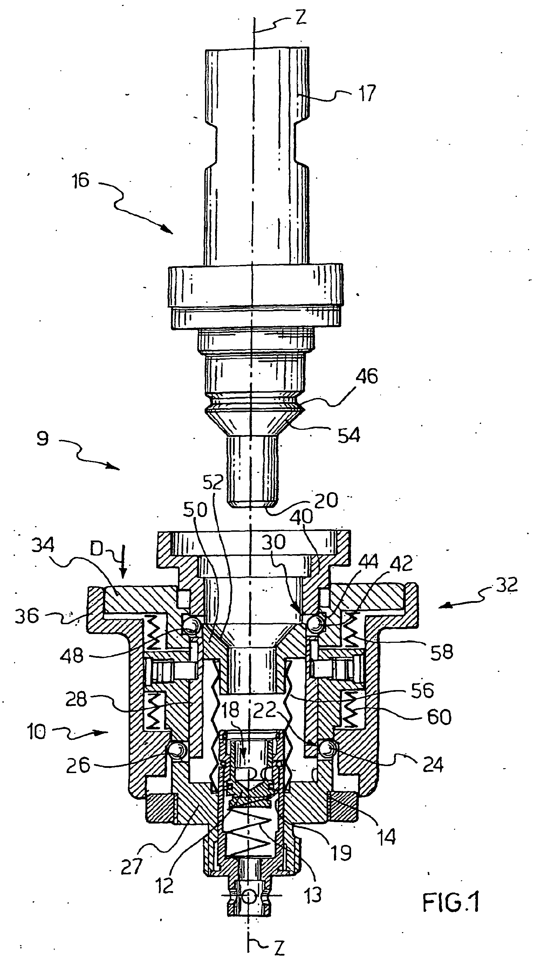

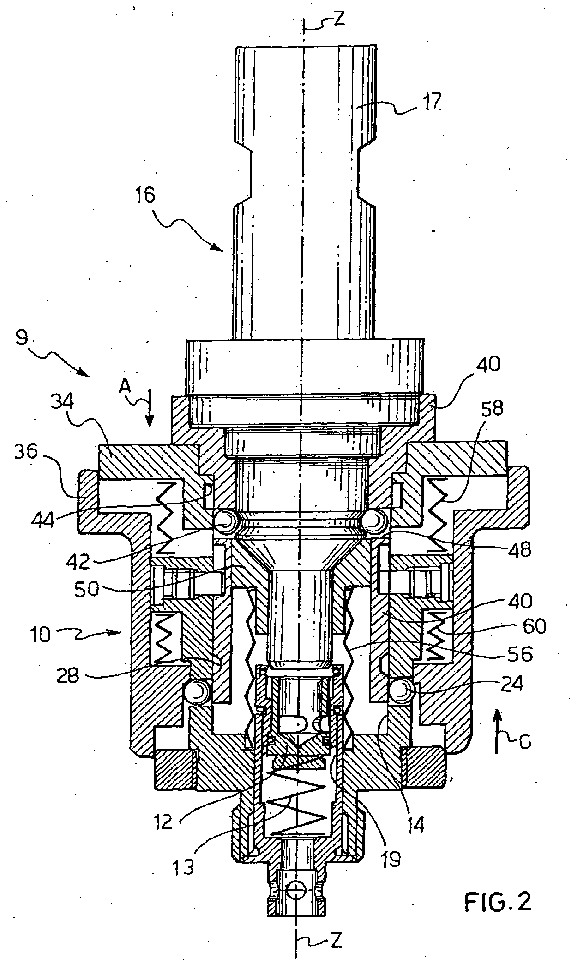

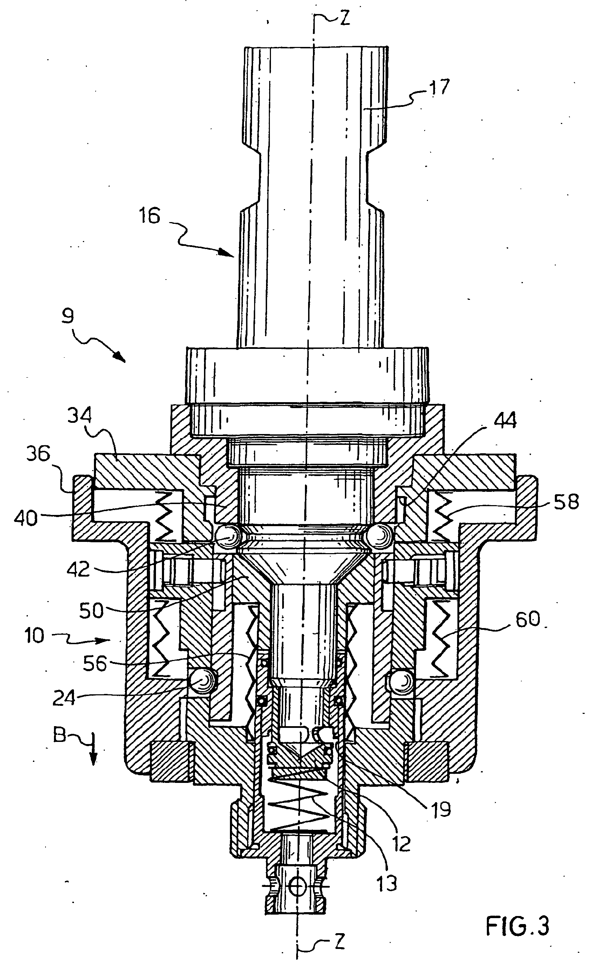

With reference to the figures, the outer bush 36, with the sleeve 40, is mobile between an upper limit stop position, which is a non-operative position, spaced from said valve seat 19 and shown in figures 1 and 2, and a lower limit stop position, which is an operative position, near to said valve seat 19 and shown in figure 3: such a lower limit stop position coincides with the aforementioned blocking and delivery position, where, together with the blocking means 22, the further blocking means 30 act and where the valve seat 19 is open. The inner bush 34 is mobile between a lower limit stop position, shown in figure 1, and an upper limit stop position, shown in figures 2 and 3: such a lower limit stop position coincides with an unblocking position of the joint 16 from the maneuvering equipment 32, where the further removable blocking means 30 are disengaged.

Such further removable blocking means 30 are, in the example of the figures, of the release type and provide, for example, a series of further balls 42 distributed circumferentially around the axis Z-Z, such further balls 42 moving in directions substantially perpendicular to the axis Z-Z between first throats 44, realized inside the inner bush 34, and second throats 46, laterally realized on -the joint 16 and where the housing of the further balls 42 is partial: when the joint 16 is in a blocked position without delivery, the further balls 42 are arranged within the first throats 44 on the inner bush 34. Since between the joint 16 and the inner bush 34 the sleeve 40 is provided, a series of radial passing openings 48 are realized therein, circumferentially arranged and acting as connection between the first throats 44 and the second throats 46.Inside the sleeve 40 a glass bell 50 is also provided, having a tubular structure able to slide axially along the sleeve 40: the bell 50 is internally shaped with a frusto-conical surface 52 corresponding to a frusto-conical side surface 54 of the joint 16, said frusto-conical surfaces 52 and 54 being leant when the joint 16 is inserted in the maneuvering equipment 32. The g-bell 50 is supported by at least one spring 56 which, when the joint 16 is removed from the maneuvering equipment 32 covers, at least partially, the port of the passing openings 48 , preventing the fall of the further balls 42 in the seat 14.

It should now be precised that, in the example of the figures, the throats 28 of the blocking means 22 of the joint in the seat 14, integral with the joint 16 itself, are realized outside the sleeve 40, at the bottom with respect to the passing openings 48.

It should be highlighted that, besides the spring 56, first and second elastic means 58 and 60 are provided, which operate axially between surfaces of the socket body 27 and opposite surfaces of the inner and outer bush 34 and 36 respectively. Such elastic elements 58 and 60 have an auxiliary function, according to provisions of the prior art and together with conventional axial guide and stop means, to realize the different positions of the maneuvering equipment 32 which have been described above.

The operation of the supplying terminal unit 9 for distribution plants of compressed gases, according to the invention, will become clearer from the following, with reference to the arrows A, B, C and D of figures 1, 2 and 3 quoted below.

An operation of attachment with delivery of the joint 16, which is initially detached from the socket 10, will now be first described.

The socket 10 is initially in the condition wherein the outer bush 36 of the maneuvering equipment 32 is in the upper limit stop position and the inner bush 34 is in the lower limit stop position (figure 1).

By inserting the joint 16 in the maneuvering equipment 32, further removable blocking means 30 are initially actuated, i.e. the inner bush 34 releases (arrow A) to the upper limit stop position, making the joint 16 integral with the sleeve 40, since the further balls 42 which insert into the second throats 46 of the joint 16 also remain partially engaged in the passing openings 48 of the sleeve 40 itself (figure 2).

By continuing to push the joint 16 towards the socket 10, the blocking means 22 are actuated, i.e. the outer bush 36 releases (arrow B) at the lower limit stop position, taking with it also the joint 16, integral with it thanks, as it has just been seen, to the sleeve 40: the lower end 20 of the joint 16 thus simultaneously actuates the opening of the valve seat 19 with the relative delivery (figure 3).

A detachment operation of the joint 16 from the socket 10 will now be described, starting from the delivery position just shown (figure 3).

To interrupt the delivery, it is necessary to lift (arrow C) the outer bush 36 up to its upper limit stop position: the free and lower end 20 of the joint 16, which is integral with the outer bush 36 thanks to the sleeve 40, lifts itself and disengages the shutter 12 which thus closes the valve seat (figure 2). In this operating condition the favorable degassing effect of the consumption piping connected to the joint 16 is also obtained.

To operate the detachment of the joint 16 from the maneuvering equipment 32, the inner bush 34 must be pressed (arrow D) up to the lower limit stop position: the further removable blocking means 30 are thus disengaged, since the further balls 42 free the second throats 46 of the joint 16 and occupy the first throats 42 of the inner bush 34 itself (figure 1).

The main advantage achieved by the supplying unit for distribution plants of compressed gases of the present invention is that it implies a high safety degree, since it is ensured the impossibility of an accidental complete detachment of the joint from the delivery position without passing into a parking step, i.e. a step where the further blocking means act. At this point it is worth recalling the fact that the two bushes, inner and outer, of the maneuvering equipment must be actuated in two opposite directions, before being able to detach the joint: it should be understood how this type of actuation makes the supplying unit unusually safe.

Another advantage of the supplying unit object of the invention is that of being able to be used, in safety, with a single hand, both in the attachment and detachment operations of the joint.

A further non-negligible advantage of the supplying unit of the present invention is that the socket is perfectly interchangeable with known sockets already installed, also as far as the encumbrances in the wall box are concerned.

The supplying terminal unit for distribution plants of compressed gases, just described, is susceptible to other variations and modifications, all within reach of the skilled in the art and, as such, covered by the scope of protection of the present invention defined in the following claims.

## Claims

1. Supplying terminal unit (9) for distribution plants of compressed gases comprising:
- a joint (16) having one end (17) associated with a consumption piping of said compressed gas, and the other end free (20),
- a socket (10) having a socket body (27) associated with a source of said compressed gas,
- a seat (14) defined in said socket body (27) and in fluid communication with said compressed gas source,
- a gas interception valve (18), including a valve seat (19) formed in said body (27), open in said seat (14), and a shutter (12) stimulated to close said valve seat (19) by elastic means (13),
**characterized in that** it comprises:
- a sleeve (40) movably guided in the seat (14) of said socket body (27), between an operative position, near to said valve seat (19) and a non-operative position, spaced from said valve seat (19),
- first removable blocking means (30) of said joint (16) in said sleeve (40), said first blocking means (30) being removable only when said sleeve (40) is in non-operative position,
- second removable blocking means (22) of said sleeve (40) in said seat (14), which act when said first blocking means (30) of said joint (16) in said sleeve (40) are active and when said sleeve (40) is in operative position, in a position wherein the free end (20) of said joint (16) is active on said shutter (12) and keeps it opening the respective valve seat (19).

2. Unit (9) according to claim 1, **characterized in that** said joint (16) is tubular and **in that** said socket (10) has axial symmetry with respect to an axis (Z-Z) and said axis (Z-Z) coincides with that of said seat (14).

3. Unit (9) according to claim 2, **characterized in that** said first removable blocking means (30) of said joint (16) in said sleeve (40) are mounted on a maneuvering equipment (32) of said socket (10); said maneuvering equipment (32) is axially mobile along the axis (Z-Z) of said socket (10) and comprises a sleeve (40), mounted coaxially to the axis (Z-Z) of the socket (10) and inside said seat (14), an inner bush (34) and an outer bush (36), mounted coaxially to the axis (Z-Z) of the socket (10) and externally around said seat (14).

4. Unit (9) according to claim 3, **characterized in that** said inner bush (34) can be displaced axially in said outer bush (36), whereas said outer bush (36) can be displaced axially with respect to the socket body (27).

5. Unit (9) according to claim 4, **characterized in that** said sleeve (40) is mounted integral with and inside said outer bush (36), the sleeve (40) also being inside the inner bush (34), said sleeve (40), at one end, constituting a support for said joint (16).

6. Unit (9) according to claim 5, **characterized in that** said outer bush (36), with said sleeve (40), is mobile between an upper limit stop position, which corresponds to the non-operative position spaced from said valve seat (19), and a lower limit stop position, which corresponds to the operative position near to said valve seat (19).

7. Unit (9) according to claim 6, **characterized in that** said inner bush (34) is mobile between a lower limit stop position and an upper limit stop position, said lower limit stop position coinciding with an unblocking position of said joint (16) from said maneuvering equipment (32), where said first removable blocking means (30) are disengaged.

8. Unit (9) according to claim 1, **characterized in that** said second removable blocking means (22) are of the release type.

9. Unit (9) according to claims 7 and 8, **characterized in that** said second blocking means (22) comprise a series of balls (24) distributed circumferentially around the axis (Z-Z), said balls (24) moving in directions substantially perpendicular to the axis (Z-Z) between recesses (26), integral with said socket body (27), and throats (28), integral with said joint (16), said balls (24) being arranged in said throats (28) integral with said joint (16) when said second blocking means (22) act.

10. Unit (9) according to claim 1, **characterized in that** said first removable blocking means (30) are of the release type.

11. Unit (9) according to claims 7 and 10, **characterized in that** said first removable blocking means (30) comprise a series of further balls (42) distributed circumferentially around the axis (Z-Z), said further balls (42) moving in directions substantially perpendicular to the axis (Z-Z) between first throats (44), realized inside said inner bush (34), and second throats 46, realized on the side of said joint (16) and where the housing of said further balls (42) is partial, said further balls (42) being arranged within said first throats (44) on said inner bush (34) when said joint (16) is in a blocked position without delivery, in said sleeve (40) a series of radial passing openings (48) being realised, arranged circumferentially and which act as connection between said first throats (44) and said second throats (46).

12. Unit (9) according to claim 11, **characterized in that** inside said sleeve (40) a bell (50) is also provided, having a tubular structure and able to slide axially along said sleeve (40), said bell (50) being internally shaped with a frusto-conical surface (52) corresponding to a frusto-conical side surface (54) of said joint (16), said frusto-conical surfaces (52, 54) being leant when said joint (16) is inserted into the maneuvering equipment (32).

13. Unit (9) according to claim 12, **characterized in that** said bell (50) is supported by at least one spring (56) which, when said joint (16) is released from the maneuvering equipment (32), at least partially covers the port of said passing openings (48), preventing the fall of said further balls (42) in said seat (14).

14. Unit (9) according to claims 9 and 13, **characterized in that** said throats (28) of said second blocking means (22) of the joint (16) in the seat (14), integral with said joint (16), are realized outside said sleeve (40), at the bottom with respect to said passing openings (48).

15. Unit (9) according to claim 13, **characterized in that** first and second elastic elements (58, 60) are provided, which operate axially between surfaces of said socket body (27) and opposite surfaces of said inner and outer bushes (34, 36) respectively.

16. Unit (9) according to claim 1, **characterized in that** said elastic means (13) include a helical spring.

## Patentansprüche

1. Versorgungsstutzeneinheit (9) für Druckgasverteilungssysteme umfassend:
- einen Stutzen(16), wobei ein Ende (17) mit einer Verbrauchsleitung des Druckgases assoziiert ist und das andere Ende (20) frei ist;
- eine Muffe (10) mit einem Muffenkörper (27), der mit einer Druckgasquelle assoziiert ist,
- einen Sitz (14) festgelegt im Muffenkörper (27) und in Flüssigverbindung mit der Druckgasquelle,
- ein Gasabfangventil (18), einschließend einen Ventilsitz (19), der im Körper (27) ausgebildet und offen im Sitz (14) ist, und einen Verschluss (12) stimuliert zum Schließen des Ventilsitzes (19) durch elastische Mittel (13);
**dadurch gekennzeichnet, dass** sie umfasst:
- eine Buchse (40), die zwischen einer Betriebsposition in der Nähe des Ventilsitzes (19) und einer Nicht-Betriebsposition im Abstand vom Ventilsitz im Sitz (14) des Muffenkörpers (27) bewegbar geführt ist,
- erste, entfernbare Blockiermittel (30) des Stutzen (16) in der Buchse (40), wobei das erste Blockiermittel (30) nur entfernbar ist, wenn die Buchse (40) in Nicht-Betriebsposition ist,
- zweite, entfernbare Blockiermittel (22) der Buchse (40) im Sitz (14), die wirken, wenn die ersten Blockiermittel (30) des Stutzen (16) in der Buchse (40) aktiv sind und wenn die Buchse (40) in Betriebsposition ist, in einer Position in der das freie Ende (20) der Verbindung (16) aktiv auf den Verschluss (12) ist und den entsprechenden Ventilsitz (19) offen hält.

2. Einheit (9) nach Anspruch 1, **dadurch gekennzeichnet, dass** der Stutzen (16) rohrförmig ist und die Muffe (10) axial-symetrisch in Bezug auf eine Achse (Z-Z) ist und die Achse (Z-Z) zusammenfällt mit der vom Sitz (14).

3. Einheit (9) nach Anspruch 2, **dadurch gekennzeichnet, dass** die ersten, entfernbaren Blockiermittel (30) des Stutzen (16) in der Buchse (40) auf einer Manövriereinrichtung (32) der Muffe (10) angebracht sind; die Manövriereinrichtung (32) entlang der Achse (Z-Z) der Muffe (10) axial beweglich ist und eine Buchse (40), die koaxial zur Achse (Z-Z) der Muffe (10) und innerhalb des Sitzes (14) angebracht ist, sowie eine innere Hülse (34) und eine äußere Hülse (36), die koaxial zur Achse (Z-Z) der Muffe (10) und extern um den Sitz (14) herum angebracht sind, umfasst.

4. Einheit (9) nach Anspruch 3, **dadurch gekennzeichnet, dass** die innere Hülse (34) in die äußere Hülse (36) axial verschoben sein kann, wobei die äußere Hülse (36) in Bezug auf den Muffenkörper (27) axial verschoben sein kann.

5. Einheit (9) nach Anspruch 4, **dadurch gekennzeichnet, dass** die Buchse (40) integriert mit und innerhalb der äußeren Buchse (36) angebracht ist, die Buchse (40) auch innerhalb der inneren Hülse (34) ist, wobei die Buchse (40) an einem Ende eine Stütze für den Stutzen (16) bildet.

6. Einheit (9) nach Anspruch 5, **dadurch gekennzeichnet, dass** die äußere Hülse (36) mit der Buchse (40) zwischen einer oberen Grenzstopposition, die zur vom Ventilsitz (19) beabstandeten Nicht-Betriebsposition korrespondiert, und einer unteren Grenzstopposition, die zur Betriebsposition in der Nähe des Ventilsitzes (19) korrespondiert, beweglich ist.

7. Einheit (9) nach Anspruch 6, **dadurch gekennzeichnet, dass** die innere Hülse (34) zwischen einer unteren Grenzstopposition und einer oberen Grenzstopposition beweglich ist, wobei die untere Grenzstopposition mit einer Entblockierposition des Stutzen (16) von der Manövriereinrichtung (32) zusammenfällt, wo die ersten, entfernbaren Blockiermittel (30) gelöst werden.

8. Einheit (9) nach Anspruch 1, **dadurch gekennzeichnet, dass** die zweiten, entfernbaren Blockiermittel (22) vom Auslösertyp sind.

9. Einheit (9) nach den Ansprüchen 7 und 8, **dadurch gekennzeichnet, dass** die zweiten Blockiermittel (22) eine Reihe von um die Achse (Z-Z) im Umfang verteilte Kugeln (24) umfassen, wobei die Kugeln (24) sich in Richtungen im Wesentlichen senkrecht zur Achse (Z-Z) zwischen Aussparungen (26) integriert mit dem Muffenkörper (27) und Halsstücken (28) integriert mit dem Stutzen (16) bewegen, wobei die Kugeln (24) integriert mit dem Stutzen (16) in den Halsstücken (28) angeordnet sind, wenn die zweiten Blockiermittel (22) wirken.

10. Einheit (9) nach Anspruch 1, **dadurch gekennzeichnet, dass** die ersten, entfernbaren Blockiermittel (30) vom Auslösertyp sind.

11. Einheit (9) nach den Ansprüchen 7 und 10, **dadurch gekennzeichnet, dass** die ersten, entfernbaren Blockiermittel (30) Reihen von weiteren, um die Achse (Z-Z) im Umfang herum verteilte Kugeln (42) umfassen, wobei die weiteren Kugeln (42) sich in Richtungen im Wesentlichen senkrecht zur Achse (Z-Z) bewegen zwischen ersten Halsstücken (44), realisiert innerhalb der inneren Hülse (34), und zweiten Halsstücken (46), realisiert auf der Seite des Stutzen (16) und wo das Gehäuse der weiteren Kugeln (42) teilweise ist, wobei die weiteren Kugeln (42) innerhalb der ersten Halsstücke (44) auf der inneren Hülse (34) angeordnet sind, wenn der Stutzen (16) in einer blockierten Position ohne Zufuhr ist, in der Buchse (40) eine Reihe von radialen Ausweichöffnungen (48) realisiert sind, die im Umfang herum angeordnet sind und als eine Verbindung zwischen den ersten Halsstücken (44) und den zweiten Halsstücken (46) wirken.

12. Einheit (9) nach Anspruch 11, **dadurch gekennzeichnet, dass** innerhalb der Buchse (40) auch eine Glocke (50) bereitgestellt ist, die eine rohrförmige Struktur besitzt und geeignet ist, axial entlang der Buchse (40) zu gleiten, wobei die Glocke (50) innen mit einer kegelstumpfförmigen Oberfläche (52) geformt ist, die zur kegelstumpfförmigen Oberfläche (54) des Stutzen (16) korrespondiert, wobei die kegelstwnpfförmigen Oberflächen (52, 54) geneigt sind, wenn der Stutzen (16) in die Manövriereinrichtung (32) eingesetzt wird.

13. Einheit (9) nach Anspruch 12, **dadurch gekennzeichnet, dass** die Glocke (50) durch zumindest eine Feder (56) gestützt ist, die zumindest teilweise die Öffnung der Ausweichöffnungen (48) abdeckt, wenn der Stutzen (16) von der Manövriereinrichtung (32) freigelassen wird, wobei verhindert wird, dass die weiteren Kugeln (42) in den Sitz (14) fallen.

14. Einheit (9) nach den Ansprüchen 9 und 13, **dadurch gekennzeichnet, dass** die Halsstücke (28) der zweiten Blockiermittel (22) des Stutzen (16) im Sitz (14) integriert mit dem Stutzen (16) außerhalb der Buchse (40) am Boden im Verhältnis zu den Ausweichöffnungen realisiert sind.

15. Einheit (9) nach dem Anspruch 13, **dadurch gekennzeichnet, dass** erste und zweite elastische Elemente (58, 60) bereitgestellt sind, die axial zwischen den Oberflächen der inneren bzw. der äußeren Hülsen (34, 36) betrieben werden.

16. Einheit (9) nach dem Anspruch 1, **dadurch gekennzeichnet, dass** die elastischen Mittel (13) eine Spiralfeder einschließen.

## Revendications

1. Embout de distribution (9) pour un système de distribution de gaz comprimé comprenant :
- un raccord (16) possédant une extrémité (17) associée avec une tuyauterie de consommation de gaz comprimé et dont l'autre extrémité est libre (20),
- une douille (10) possédant un corps de douille (27) associée avec une source dudit gaz comprimé,
- un siège (14) défini dans ledit corps de douille (27) et en communication fluidique avec ladite source de gaz comprimé,
- une soupape d'interception de gaz (18), possédant un siège de soupape (19) formé dans ledit corps (27), ouvert sur ledit siège (14) et un diaphragme (12) stimulé pour fenner ledit siège de soupape (19) par un dispositif élastique (13),
**caractérisé en ce qu'**il comprend :
- une gaine (40) guidée dans le siège (14) dudit corps de douille (27) de façon amovible, entre une position opérante proche dudit siège de soupape (19) et une position inopérante distante dudit siège de soupape (19),
- un premier dispositif de blocage amovible (30) dudit raccord (16) dans ladite gaine (40), ledit premier dispositif de blocage (30) étant amovible uniquement lorsque ladite gaine (40) est disposée dans une position inopérante,
- un deuxième dispositif de blocage amovible (22) de ladite gaine (40) dans ledit siège (14), qui agit lorsque ledit premier dispositif de blocage (30) dudit raccord (16) dans ladite gaine (40) est actif et lorsque ladite gaine (40) est disposée dans une position opérante, selon une position dans laquelle l'extrémité libre (20) dudit raccord (16) agit sur ledit diaphragme (12) et lui fait maintenir le siège de soupape respectif (19) ouvert.

2. Un embout (9) selon la revendication 1, **caractérisé en ce que** ledit raccord (16) est tubulaire et **en ce que** ladite douille (10) est axialement symétrique par rapport à un axe (Z-Z) et ledit axe (Z-Z) coïncide avec l'axe dudit siège (14).

3. Un embout (9) selon la revendication 2, **caractérisé ce que** ledit premier dispositif de blocage amovible (30) dudit raccord (16) dans ladite gaine (40) est monté sur un équipement de manoeuvre (32) de ladite douille (10) ; ledit équipement de manoeuvre (32) est axialement mobile le long de l'axe (Z-Z) de ladite douille (10) et comprend une gaine (40), montée co-axialement à l'axe (Z-Z) de la douille (10) et à l'intérieur dudit siège (14), une bague interne (34) et une bague externe (36), montées co-axialement à l'axe (Z-Z) de la douille (10) et extérieurement autour dudit siège (14) .

4. Un embout (9) selon la revendication 3, **caractérisé en ce que** ladite bague interne (34) peut être axialement déplacée dans ladite bague externe (36), alors que ladite bague externe (36) peut être axialement déplacée par rapport au corps de douille (27).

5. Un embout (9) selon la revendication 4, **caractérisé en ce que** ladite gaine (40) est intégralement montée avec et à l'intérieur de ladite bague externe (36), la gaine (40) étant également disposée à l'intérieur de la bague interne (34), une extrémité de ladite gaine (40) constituant un support pour ledit raccord (16).

6. Un embout (9) selon la revendication 5, **caractérisé en ce que** ladite bague externe (36) est amovible avec ladite gaine (40) entre une position d'arrêt de limite supérieure, qui correspond à la position inopérante distante dudit siège de soupape (19), et une position d'arrêt de limite inférieure, qui correspond à la position opérante proche dudit siège de soupape (19).

7. Un embout (9) selon la revendication 6, **caractérisé en ce que** ladite bague interne (34) est amovible entre une position d'arrêt de limite inférieure et une position d'arrêt de limite supérieure, ladite position d'arrêt de limite inférieure coïncidant avec une position de déblocage dudit raccord (16) par rapport audit équipement de manoeuvre (32), dans laquelle ledit premier dispositif de blocage amovible (30) est désengagé.

8. Un embout (9) selon la revendication 1, **caractérisé ce que** ledit deuxième dispositif de blocage amovible (22) est du type à détente.

9. Un embout (9) selon les revendications 7 et 8, **caractérisé en ce que** ledit deuxième dispositif de blocage (22) comprend une série de billes (24) distribuées autour de la circonférence de l'axe (Z-Z), lesdites billes (24) se déplaçant selon des directions substantiellement perpendiculaires à l'axe (Z-Z) entre des cavités (26) intégrales avec ledit corps de douille (27), et des gorges (28) intégrales avec ledit raccord (16), lesdites billes (24) étant disposées dans lesdites gorges (28) intégrales avec ledit raccord (16) lorsque ledit deuxième dispositif de blocage (22) agit.

10. Un embout (9) selon la revendication 1, **caractérisé en ce que** ledit premier dispositif de blocage amovible (30) est du type à détente.

11. Un embout (9) selon les revendications 7 et 10, **caractérisé en ce que** ledit premier dispositif de blocage amovible (30) comprend une série de billes supplémentaires (42) distribuées autour de la circonférence de l'axe (Z-Z), lesdites billes supplémentaires (42) se déplaçant selon des directions substantiellement perpendiculaires à l'axe (Z-Z) entre des premières gorges (44) réalisées à l'intérieur de ladite bague interne (34) et des deuxième gorges 46 réalisées sur le côté dudit raccord (16), et dans lequel lesdites billes supplémentaires (42) sont partiellement encloses, lesdites billes supplémentaires (42) étant disposées à l'intérieur desdites premières gorges (44) sur ladite bague interne (34) lorsque ledit raccord (16) est disposé dans une position bloquée sans distribution, une série d'ouvertures de passage radiales (48) étant réalisées dans ladite gaine (40), qui sont disposées en circonférence et qui agissent comme une connexion entre lesdites premières gorges (44) et lesdites deuxièmes gorges (46).

12. Un embout (9) selon la revendication 11, **caractérisé en ce qu'**un évasement (50) est également fourni à l'intérieur de ladite gaine (40), qui possède une structure tubulaire et peut glisser axialement le long dudit gaine (40), ledit évasement (50) étant intérieurement formé avec une surface obconique ou tronconique (52) correspondant à une surface latérale obconique (54) dudit raccord (16), lesdites surfaces obconiques ou tronconiques (52, 54) étant en appui lorsque ledit raccord (16) est inséré à l'intérieur de l'équipement de manoeuvre (32).

13. Un embout (9) selon la revendication 12, **caractérisé en ce que** ledit évasement (50) est maintenu par au moins un ressort (56), qui recouvre au moins partiellement l'orifice desdites ouvertures de passage (48) lorsque ledit raccord (16) est relâché de l'équipement de manoeuvre (32), empêchant la chute desdites billes supplémentaires (42) dans ledit siège (14).

14. Un embout (9) selon les revendications 9 et 13, **caractérisé en ce que** lesdites gorges 28 dudit deuxième dispositif de blocage (22) du raccord (16) dans le siège (14), intégrales avec ledit raccord (16), sont réalisées à l'extérieur de ladite gaine (40), au fond par rapport auxdites ouvertures de passage (48).

15. Un embout (9) selon la revendication 13, **caractérisé en ce qu'**un premier et un deuxième éléments élastiques (58, 60) sont fournis, qui fonctionnent axialement entre les surfaces dudit corps de douille (27) et à l'opposée des surfaces desdites bagues interne et externe (34, 36), respectivement.

16. Un embout (9) selon la revendication 1, **caractérisé en ce que** ledit dispositif élastique (13) comprend un ressort hélicoïdal.
